(19) Europäisches Patentamt
European Patent Office
Office européen des brevets

(11) **EP 4 691 489 A1**

(12) **EUROPEAN PATENT APPLICATION**
published in accordance with Art. 153(4) EPC

(43) Date of publication:
**11.02.2026 Bulletin 2026/07**

(21) Application number: 24780385.1

(22) Date of filing: **26.03.2024**

(51) International Patent Classification (IPC):
*A61K 45/00* (2006.01)   *A61K 31/444* (2006.01)
*A61K 31/4375* (2006.01)   *A61P 1/00* (2006.01)
*A61P 5/00* (2006.01)   *A61P 17/02* (2006.01)
*A61P 17/14* (2006.01)   *A61P 35/00* (2006.01)

(52) Cooperative Patent Classification (CPC):
**A61K 31/4375; A61K 31/444; A61K 45/00;
A61P 1/00; A61P 5/00; A61P 17/02; A61P 17/14;
A61P 35/00**

(86) International application number:
**PCT/JP2024/012098**

(87) International publication number:
**WO 2024/204277 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **27.03.2023 JP 2023049987**

(71) Applicant: **Scohia Pharma, Inc.
Fujisawa-shi, Kanagawa 251-8555 (JP)**

(72) Inventors:
• **SUGAMA, Jun
Fujisawa-shi, Kanagawa 251-8555 (JP)**
• **HIROSE, Hideki
Fujisawa-shi, Kanagawa 251-8555 (JP)**
• **MORITOH, Yusuke
Fujisawa-shi, Kanagawa 251-8555 (JP)**
• **WATANABE, Masanori
Fujisawa-shi, Kanagawa 251-8555 (JP)**

(74) Representative: **J A Kemp LLP
80 Turnmill Street
London EC1M 5QU (GB)**

(54) **GROWTH HORMONE SECRETION PROMOTER**

(57)    As specific embodiments of the invention, the present specification discloses a growth hormone secretion promoter containing a somatostatin receptor subtype 5 ("SSTR5") antagonist as an active ingredient, and the like.

[Fig. 1]

changes in blood growth hormone concentration (mean±standard deviation)

**Growth hormone**

**Description**

[Technical Field]

The present invention relates to embodiments of growth hormone secretion promoters and the like, containing a somatostatin receptor subtype 5 (hereinafter sometimes to be abbreviated as "SSTR5") antagonist as an active ingredient, and they are useful, for example, in the pharmaceutical field.

[Background Art]

Somatostatin is a peptide hormone secreted from the hypothalamus, pancreatic δ cells, gastrointestinal endocrine cells, and the like. It suppressively controls the secretion of various hormones such as growth hormone and adrenocorticotropic hormone through five types of somatostatin receptors (SSTR1-5), which are seven-transmembrane type G protein-coupled receptors. Currently, somatostatin analog preparations such as octreotide and pasireotide are used clinically to improve various symptoms associated with hormone oversecretion, such as acromegaly and Cushing's disease. However, because existing somatostatin analog preparations show binding affinity for all SSTR1-5 receptors, it is not always clear which SSTR activation is responsible for the suppression of the secretion of each hormone.

Among the five types of receptors, SSTR5 is a receptor mainly expressed in small intestinal endocrine cells and pancreatic β cells. It is known that binding of somatostatin suppressively controls the secretion of gastrointestinal hormones such as glucagon-like peptide-1, peptide YY, and the like, and insulin. On the other hand, it has been reported by analysis using human pituitary tumor tissue that SSTR5 is also expressed in various anterior pituitary hormone-producing cells, including growth hormone-producing cells. In these cells, however, other SSTRs are similarly expressed, and it has also been clarified that the receptor expression profiles thereof differ from cell to cell (Non Patent Literature 1). Regarding the relationship between SSTR5 and the control of growth hormone secretion, there are reports that suggest the relationship based on studies using cultured cells. However, its possibility is only shown at the cellular level, and it remains unclear how SSTR5 acts at the biological level to control the secretion of anterior pituitary hormones, including growth hormone (Non Patent Literature 2). SSTR5 knockout mice showed an increase in blood adrenocorticotropic hormone concentration, but no increase in blood concentration of insulin-like growth factor 1, whose production is enhanced by growth hormone. Therefore, it was unclear whether SSTR5 antagonists exert a growth hormone secretion-enhancing action (Non Patent Literature 3).

In addition, as mentioned above, since SSTR5 has been reported to be expressed in various anterior pituitary hormone-producing cells, there is a strong concern that SSTR5 antagonists may also induce promoted secretion of multiple anterior pituitary hormones, thereby achieving not only the desired growth hormone secretion enhancing action, but also unintended side effects based on the promoted secretion of other hormones.

Therefore, while a plurality of SSTR5 antagonists have been reported to date (e.g., Patent Literatures 1 to 22, Non Patent Literature 4), all SSTR5 antagonists are described as being useful for anti-diabetic use, anti-obesity use, anti-NASH use, and the like and no description is found that they exhibit a growth hormone secretion-promoting effect or that diseases that can be improved by promoting growth hormone secretion can be assumed to be applicable diseases. In addition, there have been no reports to date that SSTR5 antagonists have shown a growth hormone secretion-promoting effect in humans.

[Citation List]

[Patent Literature]

[Patent Literature 1]
WO 2014/142363
[Patent Literature 2]
WO 2015/052910
[Patent Literature 3]
WO 2015/064083
[Patent Literature 4]
WO 2010/056717
[Patent Literature 5]
WO 2010/129729
[Patent Literature 6]

WO 2011/146324
[Patent Literature 7]
WO 2012/024183
[Patent Literature 8]
WO 2016/205032
[Patent Literature 9]
WO 2006/094682
[Patent Literature 10]
WO 2006/128803
[Patent Literature 11]
WO 2007/025897
[Patent Literature 12]
WO 2007/110340
[Patent Literature 13]
WO 2008/000692
[Patent Literature 14]
WO 2008/019967
[Patent Literature 15]
WO 2008/031735
[Patent Literature 16]
WO 2008/122510
[Patent Literature 17]
WO 2008/145524
[Patent Literature 18]
WO 2008/148710
[Patent Literature 19]
WO 2021/113362
[Patent Literature 20]
WO 2021/113363
[Patent Literature 21]
WO 2021/113368
[Patent Literature 22]
WO 2023/125486

[Non Patent Literature]

[Non Patent Literature 1]
Journal of Molecular Endocrinology (2014), 53, R1-R19
[Non Patent Literature 2]
J Clin Endocrinol Metab, September, 2003, 88(9):4239-4245
[Non Patent Literature 3]
Mol Endocrinol, January 2003, 17(1):93-106
[Non Patent Literature 4]
European Journal of Medicinal Chemistry 264(2024)116017

[Summary of Invention]

[Technical Problem]

The problem of the present invention is to provide a novel growth hormone secretion promoter and the like, as embodiments thereof. More specifically, the present invention aims to provide, as one of the specific embodiments thereof, a growth hormone secretion promoter containing a somatostatin receptor subtype 5 ("SSTR5") antagonist as an active ingredient.

[Solution to Problem]

Under such circumstances, the present inventors have conducted intensive studies of the action shown by somatostatin relating to the secretion of various hormones and found that, as described in detail in the below-mentioned Examples, in tests using SSTR5 antagonists with high selectivity, the medicament has a clear growth hormone secretagogue action in human, and that unexpectedly does not affect secretion of other anterior pituitary hormones such as follicle-stimulating hormone, luteinizing hormone, thyroid-stimulating hormone, adrenocorticotropic hormone, prolactin, and the like, to which somatostatin is considered to contribute.

Based on the new finding, the present inventors have completed the present invention in which an SSTR5 antagonist has a growth hormone secretagogue action, and can be used for the prophylaxis or treatment of diseases and the like that can be improved by promoting secretion of growth hormone.

The present invention is described in the following by referring to specific embodiments. However, the present invention is not limited to these embodiments.

[1] A growth hormone secretion promoter, comprising an SSTR5 antagonist as an active ingredient.

[2] The growth hormone secretion promoter of the above-mentioned [1], which is an agent for the prophylaxis and/or treatment of a disease that is improved by promoting secretion of a growth hormone.

[3] A method for the prophylaxis and/or treatment of a disease that is improved by promoting secretion of a growth hormone, comprising administering an effective amount of an SSTR5 antagonist to a human or an animal in need of the administration.

[4] An SSTR5 antagonist for use in the prophylaxis and/or treatment of a disease that is improved by promoting secretion of a growth hormone.

[5] Use of an SSTR5 antagonist for the production of a medicament for the prophylaxis and/or treatment of a disease that is improved by promoting secretion of a growth hormone.

[6] The growth hormone secretion promoter of the above-mentioned [2], the prophylactic and/or treatment method of [3], the SSTR5 antagonist of [4], or the use of the SSTR5 antagonist of [5], wherein the disease that is improved by promoting secretion of a growth hormone is one or more diseases selected from pediatric growth hormone deficiency, short stature in Turner syndrome, short stature in chondrodystrophy, short stature in pediatric chronic renal failure, short stature in Prader-Willi syndrome, short stature in SGA, short stature in Noonan syndrome, adult growth hormone deficiency, cancer cachexia, short bowel syndrome, alopecia, skin wound, age-related hyposecretion of growth hormone, and infertility.

[7] The growth hormone secretion promoter of the above-mentioned [2], the prophylactic and/or treatment method of [3], the SSTR5 antagonist of [4], or the use of the SSTR5 antagonist of [5], wherein the disease that is improved by promoting secretion of a growth hormone is one or more diseases selected from pediatric growth hormone deficiency, short stature in Turner syndrome, short stature in chondrodystrophy, short stature in pediatric chronic renal failure, short stature in Prader-Willi syndrome, short stature in SGA, short stature in Noonan syndrome, and adult growth hormone deficiency.

[8] The growth hormone secretion promoter of the above-mentioned [1], which is a growth promoter for an animal (preferably, livestock animal and/or aquatic animal) (i.e., growth promoter for an animal (preferably, livestock animal and/or aquatic animal) comprising an SSTR5 antagonist as an as an active ingredient).

[9] A method for promoting growth of an animal, comprising administering an effective amount of an SSTR5 antagonist to an animal (preferably, livestock animal and/or aquatic animal) in need of the administration.

[10] An SSTR5 antagonist for use in promoting growth of an animal (preferably, livestock animal and/or aquatic animal).

[11] Use of an SSTR5 antagonist for the production of a growth promoter for an animal (preferably, livestock animal and/or aquatic animal).

[12] The growth hormone secretion promoter of the above-mentioned [1], the growth hormone secretion promoter of the above-mentioned [2], the prophylactic and/or treatment method of the above-mentioned [3], the SSTR5 antagonist of the above-mentioned [4], the use of the SSTR5 antagonist of the above-mentioned [5], the growth hormone secretion promoter of the above-mentioned [8], the growth promotion method of the above-mentioned [9], the SSTR5 antagonist of the above-mentioned [10], or the use of the SSTR5 antagonist of the above-mentioned [11], wherein the SSTR5 antagonist (preferably, SSTR5 selective antagonist) is selected from the compounds or salts thereof described in WO 2014/142363, WO 2015/052910, WO 2015/064083, WO 2010/056717, WO 2010/129729, WO 2011/146324, WO 2012/024183, WO 2016/205032, WO 2006/094682, WO 2006/128803, WO 2007/025897, WO 2007/110340, WO 2008/000692, WO 2008/019967, WO 2008/031735, WO 2008/122510, WO 2008/145524, WO 2008/148710, WO 2021/113362, WO 2021/113363, WO 2021/113368, WO 2023/125486, and European Journal of Medicinal Chemistry 264(2024)116017.

[13] The growth hormone secretion promoter of the above-mentioned [1], the growth hormone secretion promoter of the above-mentioned [2], the prophylactic and/or treatment method of the above-mentioned [3], the SSTR5 antagonist of the above-mentioned [4], the use of the SSTR5 antagonist of the above-mentioned [5], the growth hormone secretion promoter of the above-mentioned [8], the growth promotion method of the above-mentioned [9], the SSTR5 antagonist of the above-mentioned [10], or the use of the SSTR5 antagonist of the above-mentioned [11], wherein the SSTR5 antagonist (preferably, SSTR5 selective antagonist) is selected from the compounds or salts thereof described in WO 2015/052910 and WO 2015/064083.

[14] The growth hormone secretion promoter of the above-mentioned [1], the growth hormone secretion promoter of the above-mentioned [2], the prophylactic and/or treatment method of the above-mentioned [3], the SSTR5 antagonist of the above-mentioned [4], the use of the SSTR5 antagonist of the above-mentioned [5], the growth hormone secretion promoter of the above-mentioned [8], the growth promotion method of the above-mentioned [9], the SSTR5 antagonist of the above-mentioned [10], or the use of the SSTR5 antagonist of the above-mentioned [11], wherein the SSTR5 antagonist is selected from

6-(1-((2-cyclopropyl-5-ethoxy-4'-fluorobiphenyl-4-yl)methyl)piperidin-4-yl)-5-oxo-2-propyl-5,6,7,8-tetrahydro-1,6-naphthyridine-3-carboxylic acid,
6-(1-((6-cyclopropyl-3-ethoxy-2,4'-difluorobiphenyl-4-yl)methyl)piperidin-4-yl)-5-oxo-2-propyl-5,6,7,8-tetrahydro-1,6-naphthyridine-3-carboxylic acid,
6-(1-((2-cyclopropyl-5-ethoxy-2',4'-difluorobiphenyl-4-yl)methyl)piperidin-4-yl)-5-oxo-2-propyl-5,6,7,8-tetrahydro-1,6-naphthyridine-3-carboxylic acid,
6-(1-((6-cyclopropyl-3-ethoxy-2,4'-difluorobiphenyl-4-yl)methyl)piperidin-4-yl)-2-ethyl-5-oxo-5,6,7,8-tetrahydro-1,6-naphthyridine-3-carboxylic acid,
1-(1-((6-cyclopropyl-3-ethoxy-2,4'-difluorobiphenyl-4-yl)methyl)piperidin-4-yl)-3-ethyl-2-oxo-1,2-dihydropyridine-4-carboxylic acid,
1-(1-((6-cyclopropyl-2,4'-difluoro-3-isopropoxybiphenyl-4-yl)methyl)piperidin-4-yl)-3-methyl-2-oxo-1,2-dihydropyridine-4-carboxylic acid,
1-(1-((6-cyclopropyl-3-ethoxy-2,4'-difluorobiphenyl-4-yl)methyl)piperidin-4-yl)-3-methyl-2-oxo-1,2-dihydropyridine-4-carboxylic acid,
and salts thereof.

[15] The growth hormone secretion promoter of the above-mentioned [1], the growth hormone secretion promoter of the above-mentioned [2], the prophylactic and/or treatment method of the above-mentioned [3], the SSTR5 antagonist of the above-mentioned [4], the use of the SSTR5 antagonist of the above-mentioned [5], the growth hormone secretion promoter of the above-mentioned [8], the growth promotion method of the above-mentioned [9], the SSTR5 antagonist of the above-mentioned [10], or the SSTR5 antagonist of the above-mentioned [11], wherein the SSTR5 antagonist is 6-(1-((2-cyclopropyl-5-ethoxy-4'-fluorobiphenyl-4-yl)methyl)piperidin-4-yl)-5-oxo-2-propyl-5,6,7,8-tetrahydro-1,6-naphthyridine-3-carboxylic acid or a salt thereof.

In the above-mentioned embodiments [12] to [15], the preferred disease improved by a promotion of the secretion of a growth hormone includes one or more diseases selected from pediatric growth hormone deficiency, short stature in Turner syndrome, short stature in chondrodystrophy, short stature in pediatric chronic renal failure, short stature in Prader-Willi syndrome, short stature in SGA, short stature in Noonan syndrome, adult growth hormone deficiency, cancer cachexia, short bowel syndrome, alopecia, skin wound, age-related hyposecretion of growth hormone, and infertility, and the more preferred disease improved by a promotion of the secretion of a growth hormone includes one or more diseases selected from pediatric growth hormone deficiency, short stature in Turner syndrome, short stature in chondrodystrophy, short stature in pediatric chronic renal failure, short stature in Prader-Willi syndrome, short stature in SGA, short stature in Noonan syndrome, and adult growth hormone deficiency.

[16] The growth hormone secretion promoter of the above-mentioned [2], the prophylactic and/or treatment method of the above-mentioned [3], the SSTR5 antagonist of the above-mentioned [4], or the use of the SSTR5 antagonist of the above-mentioned [5], wherein the SSTR5 antagonist is 6-(1-((2-cyclopropyl-5-ethoxy-4'-fluorobiphenyl-4-yl)methyl)piperidin-4-yl)-5-oxo-2-propyl-5,6,7,8-tetrahydro-1,6-naphthyridine-3-carboxylic acid or a salt thereof, and the disease that is improved by promoting secretion of a growth hormone is one or more diseases selected from pediatric growth hormone deficiency, short stature in Turner syndrome, short stature in chondrodystrophy, short stature in pediatric chronic renal failure, short stature in Prader-Willi syndrome, short stature in SGA, short stature in Noonan syndrome, adult growth hormone deficiency, cancer cachexia, short bowel syndrome, alopecia, skin wound, hyposecretion of age-related growth hormone, and infertility.

[17] The growth hormone secretion promoter of the above-mentioned [2], the prophylactic and/or treatment method of [3], the SSTR5 antagonist of [4], or the use of the SSTR5 antagonist of [5], wherein the SSTR5 antagonist is 6-(1-((2-cyclopropyl-5-ethoxy-4'-fluorobiphenyl-4-yl)methyl)piperidin-4-yl)-5-oxo-2-propyl-5,6,7,8-tetrahydro-1,6-naphthyridine-3-carboxylic acid or a salt thereof, and the disease that is improved by promoting secretion of a growth hormone is one or more diseases selected from pediatric growth hormone deficiency, short stature in Turner syndrome, short stature in chondrodystrophy, short stature in pediatric chronic renal failure, short stature in Prader-Willi syndrome, short stature in SGA, short stature in Noonan syndrome, and adult growth hormone deficiency.

[Advantageous Effects of Invention]

The present invention provides novel growth hormone secretion promoters and the like, as an embodiment thereof. More specifically, the present invention provides, as one of the specific embodiments thereof, a growth hormone secretion promoter containing a somatostatin receptor subtype 5 ("SSTR5") antagonist as an active ingredient. The growth hormone secretion promoter is expected to afford a selective growth hormone secretagogue action, without substantially affecting secretion of other anterior pituitary hormones (all or some of follicle-stimulating hormone, luteinizing hormone, thyroid-stimulating hormone, adrenocorticotropic hormone, prolactin, and the like) involving somatostatin.

[Brief Description of Drawings]

[Fig. 1]

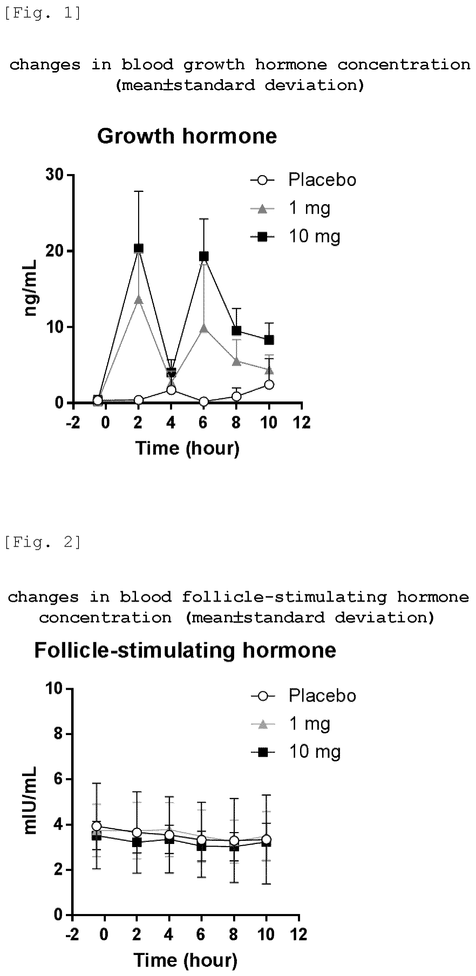

Fig. 1 shows changes over time in blood growth hormone concentration evaluated in Example 3 described later.
[Fig. 2]
Fig. 2 shows changes over time in blood follicle-stimulating hormone concentration evaluated in Example 3 described later.
[Fig. 3]

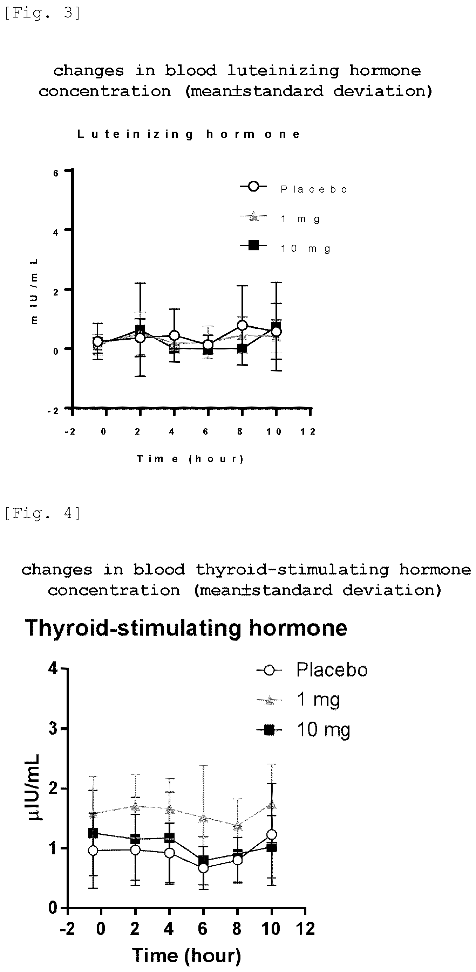

Fig. 3 shows changes over time in blood luteinizing hormone concentration evaluated in Example 3 described later.
[Fig. 4]
Fig. 4 shows changes over time in blood thyroid-stimulating hormone concentration evaluated in Example 3 described later.
[Fig. 5]

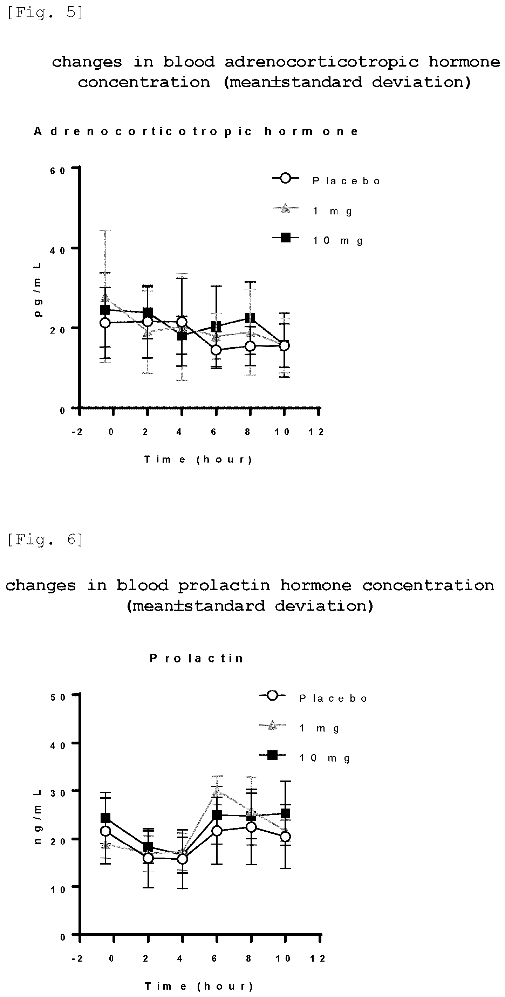

Fig. 5 shows changes over time in blood adrenocorticotropic hormone concentration evaluated in Example 3 described later.
[Fig. 6]
Fig. 6 shows changes over time in blood prolactin concentration evaluated in Example 3 described later.

[Description of Embodiments]

In the present specification, the above-mentioned embodiments are disclosed such as a growth hormone secretion promoter containing an SSTR5 antagonist as an active ingredient, and the growth hormone secretion promoter which is an agent for the prophylaxis and/or treatment of a disease that is improved by promoting secretion of a growth hormone; a method for the prophylaxis and/or treatment of a disease that is improved by promoting secretion of a growth hormone, including administering an effective amount of an SSTR5 antagonist to a human or an animal in need of the administration; an SSTR5 antagonist for use in the prophylaxis and/or treatment of a disease that is improved by promoting secretion of a growth hormone; use of an SSTR5 antagonist for the production of a medicament for the prophylaxis and/or treatment of a disease that is improved by promoting secretion of a growth hormone; and the like.

The present invention is described in detail below according to the embodiments thereof. Unless otherwise specified in the text, all technical terms and scientific terms used in the present specification have the same meaning as those generally understood by a person skilled in the art to which the present invention pertains.

(SSTR5 antagonist)

The somatostatin receptor is one of the seven transmembrane type G protein-coupled receptors, and five subtypes have been discovered to date, respectively named SSTR1, SSTR2, SSTR3, SSTR4, and SSTR5. In the present specification, the "SSTR5 antagonist" refers to all compounds that have an antagonistic action on SSTR5. Many "SSTR5 antagonists" are already known in the art, and the "SSTR5 antagonist" is recognized and widely used by those of ordinary skill in the art as a comprehensive technical term referring to these groups of compounds.

In the embodiments of the present invention, those of ordinary skill in the art can appropriately select a preferred SSTR5 antagonist from the aspects of effectiveness, safety and the like. In particular, among the five SSTRs, it is preferable to select one (SSTR5 selective antagonist) that has high selectivity for SSTR5 (SSTR5 selective antagonistic action). Such SSTR5 antagonistic action and selectivity for SSTR5 can be confirmed according to methods known in the art.

Examples of such SSTR5 antagonist include the compounds or salts thereof described in WO 2014/142363, WO 2015/052910, WO 2015/064083, WO 2010/056717, WO 2010/129729, WO 2011/146324, WO 2012/024183, WO 2016/205032, WO 2006/094682, WO 2006/128803, WO 2007/025897, WO 2007/110340, WO 2008/000692, WO 2008/019967, WO 2008/031735, WO 2008/122510, WO 2008/145524, WO 2008/148710, WO 2021/113362, WO 2021/113363, WO 2021/113368, and WO 2023/125486, and European Journal of Medicinal Chemistry 264(2024)116017.

Among these, preferred specific compounds include

6-(1-((2-cyclopropyl-5-ethoxy-4'-fluorobiphenyl-4-yl)methyl)piperidin-4-yl)-5-oxo-2-propyl-5,6,7,8-tetrahydro-1,6-naphthyridine-3-carboxylic acid (compound described in WO 2015/052910, Example 4) or a salt thereof,

6-(1-((6-cyclopropyl-3-ethoxy-2,4'-difluorobiphenyl-4-yl)methyl)piperidin-4-yl)-5-oxo-2-propyl-5,6,7,8-tetrahydro-1,6-naphthyridine-3-carboxylic acid (compound described in WO 2015/052910, Example 67),

6-(1-((2-cyclopropyl-5-ethoxy-2',4'-difluorobiphenyl-4-yl)methyl)piperidin-4-yl)-5-oxo-2-propyl-5,6,7,8-tetrahydro-1,6-naphthyridine-3-carboxylic acid (compound described in WO 2015/052910, Example 35),

6-(1-((6-cyclopropyl-3-ethoxy-2,4'-difluorobiphenyl-4-yl)methyl)piperidin-4-yl)-2-ethyl-5-oxo-5,6,7,8-tetrahydro-1,6-naphthyridine-3-carboxylic acid (compound described in WO 2015/052910, Example 74),

1-(1-((6-cyclopropyl-3-ethoxy-2,4'-difluorobiphenyl-4-yl)methyl)piperidin-4-yl)-3-ethyl-2-oxo-1,2-dihydropyridine-4-carboxylic acid (compound described in WO 2015/064083, Example 44),

1-(1-((6-cyclopropyl-2,4'-difluoro-3-isopropoxybiphenyl-4-yl)methyl)piperidin-4-yl)-3-methyl-2-oxo-1,2-dihydropyridine-4-carboxylic acid (compound described in WO 2015/064083, Example 49),

1-(1-((6-cyclopropyl-3-ethoxy-2,4'-difluorobiphenyl-4-yl)methyl)piperidin-4-yl)-3-methyl-2-oxo-1,2-dihydropyridine-4-carboxylic acid (compound described in WO 2015/064083, Example 50),

4-{8-[(2-(cyclopropyl)-5-trifluoromethylphenyl)methyl]-2-oxo-1-oxa-3,8-diazaspiro[4.5]dec-3-yl}benzoic acid (compound described in WO 2012/024183, Example 4-50),

6-{8-[(4-ethoxy-2',3',4'-trifluorobiphenyl-2-yl)methyl]-1-oxa-2,8-diazaspiro[4.5]dec-2-en-3-yl}pyridine-3-carboxylic acid (compound described in WO 2011/146324, Example 30),

4-[8-[(2-cyclopropyl-5-ethoxy-4-methyl-phenyl)methyl]-2-oxo-1,3,8-triazaspiro[4.5]decan-3-yl]benzoic acid (compound described in WO 2016/205032, Example 1),

N-[1-(2,6-diethoxy-4'-fluoro-biphenyl-4-ylmethyl)-piperidin-4-yl]-5-methyl-nicotine amide (compound described in WO 2006/128803, Example 153),

6-[1-(4-chloro-3,5-diethoxy-benzyl)-piperidin-4-ylamino]-nicotinic acid (compound described in WO 2008/019967, Example 70), and

4-(8-((2-cyclopropyl-5-ethoxy-4'-fluoro-[1,1'-biphenyl]-4-yl)methyl)-2-oxo-1-oxa-3,8-diazaspiro[4.5]decan-3-yl) benzenesulfonic acid (compound described in WO 2021/113362, Example 1), and

4-(7-(2-cyclopropyl-5-ethoxy-4-methylbenzyl)-2,7-diazaspiro[3.5]non-2-yl)-2-fluorobenzoic acid (compound described in WO 2023/125486, Example 34), 4-(((1-((2,6-diethoxy-4'-fluoro-[1,1'-biphenyl]-4-yl)methyl)-4-fluoropiperidin-4-yl)methyl)amino)-2-methoxybenzoic acid (compound described in European Journal of Medicinal Chemistry 264(2024)116017 as compound 23),

and salts thereof.

Furthermore, particularly preferred specific compound includes

6-(1-((2-cyclopropyl-5-ethoxy-4'-fluorobiphenyl-4-yl)methyl)piperidin-4-yl)-5-oxo-2-propyl-5,6,7,8-tetrahydro-1,6-naphthyridine-3-carboxylic acid,

6-(1-((6-cyclopropyl-3-ethoxy-2,4'-difluorobiphenyl-4-yl)methyl)piperidin-4-yl)-5-oxo-2-propyl-5,6,7,8-tetrahydro-1,6-naphthyridine-3-carboxylic acid,

6-(1-((2-cyclopropyl-5-ethoxy-2',4'-difluorobiphenyl-4-yl)methyl)piperidin-4-yl)-5-oxo-2-propyl-5,6,7,8-tetrahydro-1,6-naphthyridine-3-carboxylic acid,

6-(1-((6-cyclopropyl-3-ethoxy-2,4'-difluorobiphenyl-4-yl)methyl)piperidin-4-yl)-2-ethyl-5-oxo-5,6,7,8-tetrahydro-1,6-naphthyridine-3-carboxylic acid,

1-(1-((6-cyclopropyl-3-ethoxy-2,4'-difluorobiphenyl-4-yl)methyl)piperidin-4-yl)-3-ethyl-2-oxo-1,2-dihydropyridine-4-carboxylic acid,

1-(1-((6-cyclopropyl-2,4'-difluoro-3-isopropoxybiphenyl-4-yl)methyl)piperidin-4-yl)-3-methyl-2-oxo-1,2-dihydropyridine-4-carboxylic acid,

1-(1-((6-cyclopropyl-3-ethoxy-2,4'-difluorobiphenyl-4-yl)methyl)piperidin-4-yl)-3-methyl-2-oxo-1,2-dihydropyridine-4-carboxylic acid,

and salts thereof.

Most preferred specific compound is, for example, 6-(1-((2-cyclopropyl-5-ethoxy-4'-fluorobiphenyl-4-yl)methyl)piperidin-4-yl)-5-oxo-2-propyl-5,6,7,8-tetrahydro-1,6-naphthyridine-3-carboxylic acid or a salt thereof.

Those of ordinary skill in the art can produce the above-mentioned "SSTR5 antagonists" by appropriately utilizing the methods described in the above-mentioned Patent Literatures and the methods known in the art, and use them in the embodiments of the present invention.

As demonstrated using representative SSTR5 antagonists in the Examples section described below, the "SSTR5 antagonist" exerts the desired growth hormone secretagogue action based on the antagonistic action thereof on SSTR5.

In the embodiments of the present invention, the "SSTR5 antagonist" can be used either in a free form or in the form of a salt thereof (preferably a pharmaceutically acceptable salt thereof). Those skilled in the art can appropriately select from the both forms and carry out the present invention based on the characteristics of each SSTR5 antagonist used.

Examples of salt acceptable as the medicament include salts with acid such as salts with inorganic acid such as hydrochloride, hydrobromide, sulfate, phosphate, and the like, salts with organic acid such as acetate, fumarate, oxalate, citrate, methanesulfonate, benzenesulfonate, tosylate, maleate, and the like; and salts with base such as alkali metal salts such as sodium salt, potassium salt, and the like, alkaline earth metal salts such as calcium salt and the like, and the like; salts with amino acid such as glycinate, lysinate, argininate, ornithinate, glutamate, aspartate, and the like.

In the embodiments of the invention, SSTR5 antagonists can also be used in the form of prodrugs thereof. Those of ordinary skill in the art can appropriately design the prodrug, and the prodrug can be produced by a method known per se. The prodrug may also be one that transforms into the SSTR5 antagonist of interest under physiological conditions, as described in "Molecular Design", pp. 163-198 in "Development of Pharmaceuticals", Vol. 7, published by Hirokawa Shoten 1990.

(Use)

(1) Use as medicaments

The present inventors have conducted intensive studies and found for the first time that SSTR5 antagonists afford a superior growth hormone secretagogue action by the SSTR5 antagonistic action thereof. Due to the growth hormone secretagogue action, SSTR5 antagonists can promote growth hormone secretion from the pituitary gland and improve pathological conditions caused by growth hormone insufficiency, in diseases caused by the hyposecretion of growth hormones and/or diseases in which growth hormone is relatively insufficient and can be expected to be improved by supplementation thereof (e.g., diseases for which growth hormone replacement therapy is applied). Therefore, SSTR5 antagonists are useful for the prophylaxis and/or treatment of diseases that can be improved by promoting secretion of growth hormone in humans or animals (particularly in humans). Such diseases (particularly diseases in humans) that can be improved by promoting secretion of growth hormone include, but are not limited to, the following diseases and symptoms.

(Disease examples)

Pediatric growth hormone deficiency, short stature in Turner syndrome, short stature in chondrodystrophy, short stature in pediatric chronic renal failure, short stature in Prader-Willi syndrome, short stature in SGA, short stature in Noonan syndrome, adult growth hormone deficiency, cancer cachexia, short bowel syndrome, alopecia, skin wound (e.g., trauma, burn, pressure ulcer, diabetic foot, etc.), age-related hyposecretion of growth hormone (e.g., bone mass decrease, muscle mass decrease, body fats increase, lipid metabolic abnormality, fatty liver, fatigability, poor concentration, listlessness, emotional lability, drying and/or thinning of skin, etc., each of which is age-related), and infertility; and the like.

In this embodiment, for example, pediatric growth hormone deficiency, short stature in Turner syndrome, short stature in chondrodystrophy, short stature in pediatric chronic renal failure, short stature in Prader-Willi syndrome, short stature in SGA, short stature in Noonan syndrome, adult growth hormone deficiency, cancer cachexia, short bowel syndrome, alopecia, skin wound (e.g., trauma, burn, pressure ulcer, diabetic foot, etc.), and age-related hyposecretion of growth hormone (e.g., bone mass decrease, muscle mass decrease, body fats increase, lipid metabolic abnormality, fatty liver, fatigability, poor concentration, listlessness, emotional lability, drying and/or thinning of skin, etc., each of which is age-related) are preferred, and pediatric growth hormone deficiency, short stature in Turner syndrome, short stature in chondrodystrophy, short stature in pediatric chronic renal failure, short stature in Prader-Willi syndrome, short stature in SGA, short stature in Noonan syndrome, and adult growth hormone deficiency are more preferred.

In the present specification, the "prophylaxis" includes preventing the onset of a disease (all pathological conditions or one or more pathological conditions or symptoms) and delaying the onset of the disease. A "prophylactically effective amount" refers to a dose of SSTR5 antagonist sufficient to achieve such purposes.

In the present specification, the "treatment" includes curing a disease (all pathological conditions or symptoms or one or more pathological conditions or symptoms), improving the disease, and suppressing the progression of the severity of the disease. A "therapeutically effective amount" refers to a dose of SSTR5 antagonist sufficient to achieve such purposes.

(2) Use as veterinary growth promoter

As described above, since SSTR5 antagonists afford a superior growth hormone secretagogue action by the SSTR5 antagonistic action thereof, they can also be used as veterinary growth promoters (fattening agent, etc.).

(Administration form)

In the embodiments of the present invention, the SSTR5 antagonist can be used either alone or in the form of a pharmaceutical composition containing the SSTR5 antagonist as an active ingredient together with a pharmaceutically acceptable carrier.

Examples of such pharmaceutical composition include tablets (including sugar-coated tablet, film-coated tablet, sublingual tablet, orally disintegrating tablet, buccal tablet, and the like), pill, powder, granule, capsules (including soft capsule, and microcapsule), troche, syrup, liquid, emulsion, suspension, controlled-release preparations (e.g., immediate-release preparation, sustained-release preparation, sustained-release microcapsule), aerosols, films (e.g., orally disintegrable films, and mouth cavity mucosa patching film), injections (e.g., subcutaneous injection, intravenous injections (e.g., bolus), intramuscular injection, and intraperitoneal injection), drip transfusion, transdermal absorption type preparation, ointment, lotion, adhesive preparation, suppositories (e.g., rectal suppository, vaginal suppository), pellet, nasal preparation, pulmonary preparation (inhalant), eye drop, and the like.

In the present specification, as the "pharmaceutically acceptable carrier", various carriers conventionally used in the field of pharmaceutical technology can be used.

As the specific examples of the "pharmaceutically acceptable carrier", in solid preparations, excipients (e.g., lactose, sucrose, D-mannitol, starch, cornstarch, crystalline cellulose, light anhydrous silicic acid, etc.), lubricants (e.g., magnesium stearate, talc, colloid silica, etc.), binders (e.g., crystalline cellulose, sucrose, D-mannitol, dextrin, hydroxypropylcellulose, hydroxypropylmethylcellulose, polyvinylpyrrolidone, starch, sucrose, gelatin, methylcellulose, sodium carboxymethylcellulose, etc.), disintegrants (e.g., starch, carboxymethylcellulose, carboxymethylcellulose calcium, sodium carboxymethyl starch, L-hydroxypropylcellulose, etc.), and the like can be used.

In liquid preparations, solvents (e.g., water for injection, isotonic brine, alcohol, propylene glycol, macrogol, sesame oil, etc.), solubilizing agents (e.g., polyethylene glycol, propylene glycol, D-mannitol, benzoic acid benzyl, ethanol, triethanolamine, sodium carbonate, sodium citrate, etc.), suspending agents (e.g., surfactants such as stearyltriethanolamine, sodium lauryl sulfate, lauryl aminopropionic acid, lecithin, benzalkonium chloride, glycerol monostearate, and the like; hydrophilic polymers such as poly(vinyl alcohol), polyvinylpyrrolidone, sodium carboxymethylcellulose, methylcellulose, hydroxymethylcellulose, hydroxypropylcellulose, and the like; and the like), isotonicity agents (e.g., glucose, D-sorbitol, sodium chloride, glycerol, D-mannitol, etc.), buffering agents (e.g., buffer of phosphate, citrate, or the like, etc.), and soothing agents (e.g., benzyl alcohol etc.), and the like can be used.

Where necessary, formulation additives such as antiseptics (e.g., paraoxybenzoic acid esters, chlorobutanol, benzyl alcohol, sorbic acid, etc.), antioxidants (e.g., sulfite, ascorbic acid, $\alpha$-tocopherol, etc.), colorants, sweeteners, and the like may be further added.

The above-mentioned pharmaceutical composition generally containing an SSTR5 antagonist in an amount of 0.01 to 99% (w/w), preferably 0.1 to 85% (w/w), of the total amount of the preparation, can be produced, though the amount varies depending on the dosage form, administration method, carrier, and the like. The pharmaceutical composition can be produced by methods conventionally used in the field of pharmaceutical technology. The pharmaceutical composition may be formulated into a controlled-release preparation, such as immediate-release preparation, sustained-release preparation, and the like, containing the active ingredient.

When used as a veterinary growth promoter, the SSTR5 antagonist can be administered to an animal in need thereof by using a general use form in animal breeding. It is also possible, for example, to administer the SSTR5 antagonist alone or as a mixture with animal feed or water.

(Subject of administration)

The SSTR5 antagonist used in the embodiment of the present invention is expected to have low toxicity and few side effects, and also has superior properties as a pharmaceutical product. Therefore, SSTR5 antagonist can be safely administered to humans or animals (e.g., livestock animals (bovine, swine, horse, sheep, chicken, etc.), aquatic animals (tuna, porgy, salmon, trout, adult yellowtail, toadfish, Epinephelus bruneus, etc.) and the like) (particularly, humans).

(Administration route)

In the embodiments of the present invention, an SSTR5 antagonist may be administered orally or parenterally (e.g., intravenously, by drip, intramuscularly, subcutaneously, viscerally, intranasally, intradermally, transdermally, ophthalmically, intracerebrally, rectally, intravaginally, intraperitoneally, intralesionally) alone or as a pharmaceutical composition.

(Dose)

The dose of the SSTR5 antagonist varies depending on the subject of administration, administration route, and the age and symptoms of the subject, and is not particularly limited. Those of ordinary skill in the art can determine the dose as appropriate.

For example, in the case of administration to humans, per one administration, the dose is, for example, 0.1 to 320 mg, preferably 1 to 160 mg, per oral administration of the SSTR5 antagonist as the active ingredient. A more preferred dose is 1 to 80 mg (more specific dose is, for example, 1 mg, 3 mg, 10 mg, 20 mg, 40 mg, or 80 mg; another specific dose is, for example, 15 mg, 25 mg, 35 mg, or 75 mg). The dose can be administered in 1 to 3 divided doses per day. In addition, in the case of administration in the form of a sustained-release preparation, it can also be administered every other day or at longer intervals so as to correspond to the dose.

(Combination use with other agents)

In the embodiment of the present invention, the SSTR5 antagonist can be combined with other medicaments and used for the prophylaxis or treatment of a target disease, and superior prophylactic and/or therapeutic effect by the combined use with other medicaments can be expected. It is also expected that such combination therapy will lower the dose of other medicaments and reduce the side effects they have. Such combined use with other medicaments is also included in the scope of the present invention.

The medicaments that can be used in combination with such SSTR5 antagonists (hereinafter sometimes to be abbreviated as concomitant drug) can be selected as appropriate, taking into account the type of disease of the patient, the severity of the symptoms thereof, and the like. For example, combined use with growth hormone preparations (somatropin, somapacitan, somatrogon, lonapegsomatropin, PEG-somatropin, etc.), ghrelin-like acting drugs (anamorelin, LUM-201, etc.), human C-type sodium diuretic peptide preparations (vosoritide, etc.), glucagon-like peptide-2 preparations (teduglutide, etc.), follicle-stimulating hormone preparations (follitropin alfa, corifollitropin alfa, etc.), minoxidil, or finasteride is considered.

The administration form of the concomitant drug is not particularly limited, and the SSTR5 antagonist and the concomitant drug may be combined at the time of administration. For example, they can be used in the forms of (1) administration of a preparation containing the SSTR5 antagonist and the concomitant drug in combination, (2) simultaneous or separate administration of two kinds of preparations of the SSTR5 antagonist and the concomitant drug, which have been separately formulated, by the same administration route, (3) simultaneous or separate administration of two kinds of preparations of the SSTR5 antagonist and the concomitant drug, which have been separately formulated, by different administration routes, and the like. A preferred form can be selected as appropriate depending on the actual situation in the medical settings.

A preparation containing a combination of the above-mentioned SSTR5 antagonist and a concomitant drug can be appropriately produced by those skilled in the art in accordance with the pharmaceutical composition containing the SSTR5 antagonist described above.

The dose of the concomitant drug can be appropriately selected with the dose used clinically as the standard. Furthermore, the mixing ratio of the SSTR5 antagonist and the concomitant drug can be appropriately selected according to the disease and symptoms of the subject of administration, administration route, the type of the concomitant drug to be used, and the like. Generally, it can be determined as appropriate using the general clinical dose of the concomitant drug to be used as the standard and according to the actual situation in the medical settings.

[Example]

The embodiments of the present invention are explained in more detail in the following by referring to Examples. These Examples do not limit the scope of the present invention in any manner. The reagents, apparatuses, materials and the like used in the present invention are commercially available or those of ordinary skill in the art can prepare them as appropriate, unless particularly indicated.

Example 1: Evaluation of human SSTR5 antagonist activity using intracellular cAMP concentration as an indicator

The intracellular cAMP concentration was measured using HTRF cAMP dynamic 2 kit (Cisbio). A test compound diluted with an assay buffer (5 mM HEPES (pH 7.5) (Invitrogen), 0.1% fatty-acid free BSA (Sigma), and 500 $\mu$M IBMX (Wako)-containing HBSS (Invitrogen)) was added at 2 $\mu$L/well to a 384-well white plate (Greiner) to a final concentration of 1 $\mu$M. Frozen stock of CHO (dhfr-) cells stably expressing the human SSTR5 gene (Accession No. NM_001053) was thawed in a thermostatic tank at 37°C and suspended in a passage medium (MEM alpha (Wako)) containing 10% dialyzed serum (Gemini) and 50 $\mu$g/mL gentamicin (Invitrogen). The cell suspension was centrifuged, and the cells were resuspended in an assay buffer, and added by 2 $\mu$L to each well at about 4,000 cells/well. The compound and the cells were incubated for 15 min, an assay buffer containing a final concentration 0.1 nM of somatostatin 28 (Toray Research Center) and 0.3 $\mu$M forskolin (Wako) was added by 2 $\mu$L/well, and the cells were incubated at room temperature for 30 min. cAMP-d2 and anti-cAMP-cryptate were each added by 3 $\mu$L/well, allowed to stand at room temperature for 60 min, and the fluorescence resonance energy transfer (FRET) intensity was measured using the Multi-label reader Envision (Perkin Elmer). The FRET intensity of the wells to which the test compound group was added was converted into cAMP concentration by using a calibration curve created from the FRET intensities of well groups in which cAMP at any concentration was added to the assay buffer. The inhibitory activity of the compound was calculated using the following formula.

$$\texttt{Inhibitory activity (\%) = (C-B)/(A-B) x 100}$$

A: cAMP concentration calculated from wells containing 0.3 $\mu$M forskolin
B: cAMP concentration calculated from wells containing 0.3 $\mu$M forskolin, 0.1 nM somatostatin 28
C: cAMP concentration of wells containing 0.3 $\mu$M forskolin, 0.1 nM somatostatin 28, and 1 $\mu$M test compound

The inhibition rate (%) of the test compound against SSTR5 at 1 $\mu$M is shown in Table 1.

[Table 1-1]

| test compound | compound name | inhibitory rate (%) against SSTR5 at 1 $\mu$M |
|---|---|---|
| 1 | 6-(1-((2-cyclopropyl-5-ethoxy-4'-fluorobiphenyl-4-yl)methyl) piperidin-4-yl)-5-oxo-2-propyl-5,6,7,8-tetrahydro-1,6-naphthyridine-3-carboxylic acid | 87 |
| 2 | 6-(1-((6-cyclopropyl-3-ethoxy-2,4'-difluorobiphenyl-4-yl) methyl)piperidin-4-yl)-5-oxo-2-propyl-5,6,7,8-tetrahydro-1,6-naphthyridine-3-carboxylic acid | 85 |
| 3 | 6-(1-((2-cyclopropyl-5-ethoxy-2',4'-difluorobiphenyl-4-yl) methyl)piperidin-4-yl)-5-oxo-2-propyl-5,6,7,8-tetrahydro-1,6-naphthyridine-3-carboxylic acid | 125 |

[Table 1-2]

| test compound | compound name | inhibitory rate (%) against SSTR5 at 1 $\mu$M |
|---|---|---|
| 4 | 6-(1-((6-cyclopropyl-3-ethoxy-2,4'-difluorobiphenyl-4-yl) methyl)piperidin-4-yl)-2-ethyl-5-oxo-5,6,7,8-tetrahydro-1,6-naphthyridine-3-carboxylic acid | 95 |
| 5 | 1-(1-((6-cyclopropyl-3-ethoxy-2,4'-difluorobiphenyl-4-yl) methyl)piperidin-4-yl)-3-ethyl-2-oxo-1,2-dihydropyridine-4-carboxylic acid | 87 |
| 6 | 1-(1-((6-cyclopropyl-2,4'-difluoro-3-isopropoxybiphenyl-4-yl) methyl)piperidin-4-yl)-3-methyl-2-oxo-1,2-dihydropyridine-4-carboxylic acid | 99 |

(continued)

| test compound | compound name | inhibitory rate (%) against SSTR5 at 1 μM |
|---|---|---|
| 7 | 1-(1-((6-cyclopropyl-3-ethoxy-2,4'-difluorobiphenyl-4-yl) methyl)piperidin-4-yl)-3-methyl-2-oxo-1,2-dihydropyridine-4-carboxylic acid | 87 |

As is clear from Table 1, the present test compound showed a superior SSTR5 antagonistic action.

Example 2 Evaluation of selectivity for human SSTR subtype

The selectivity of test compounds for human SSTR subtypes was investigated by radiolabeled ligand binding assay. The membrane fraction of CHO-K1 cells expressing human SSTR1-5 was suspended in am assay buffer (25 mM HEPES (pH 7.4), 5 mM MgCl$_2$, 1 mM CaCl$_2$, 10 μg/mL saponin, 0.5% fatty-acid free BSA) and incubated for 1 hr in the presence of 1 μM test compound 1 and [$^{125}$I] Tyr[11]-somatostatin 14 (Perkin Elmer). Thereafter, the solution containing the membrane fraction was transferred onto a filter plate, and after washing the filter six times with a wash buffer (25 mM HEPES, 5 mM MgCl$_2$, 1 mM CaCl$_2$), 50 μL of Microscint 20 (Packard) was added, and the radioactivity was measured using a TopCount (Perkin Elmer). The results of competitive activity of the test compound against the binding of the human SSTR subtypes with the labeled ligands are shown in Table 2.

[Table 2]

|  | test compound 1 |
|---|---|
| human SSTR subtype | labeled ligand binding rate (%) at 1 μM |
| SSTR 1 | 105.83 |
| SSTR 2 | 100.31 |
| SSTR 3 | 99.22 |
| SSTR 4 | 109.00 |
| SSTR 5 | 4.51 |

As is clear from Table 2, it was shown that test compound 1 selectively binds to SSTR5 among the human SSTR subtypes.
As shown in Example 3 described later, test compound 1 affords a very superior growth hormone secretagogue action. For this reason, as described above, it is considered preferable to use an antagonist with high selectivity for SSTR5 (SSTR5 selective antagonist) when practicing the present invention. The desired degree of selectivity for SSTR5 varies depending on the specific compound to be used and cannot be uniformly determined; however, those skilled in the art can appropriately select the selectivity by using, as one guideline, the absence of a substantial action of the compound on other SSTRs at a dose at which it exhibits the desired antagonizing effect on SSTR5. The degree of selectivity for human SSTR subtype can be evaluated, for example, by the method described in the present Example 2.

Example 3: Evaluation of growth hormone secretion enhancing action by single dose administration of test compound 1 in human

The examples in this section show the measurement results of blood anterior pituitary hormone concentrations using blood samples from a dose escalation study targeting healthy Japanese adult men, from A Randomized, Double-Blind, Placebo-Controlled, Phase 1 Study (jRCT2051210027) targeting healthy adults, which was conducted to examine the safety and tolerability, pharmacokinetics, and pharmacodynamic action of a single administration of test compound 1.

Test outline:

In a randomized, double-blind, placebo-controlled, parallel-group comparison, dose-escalation study consisting of 7 cohorts, a single dose of test compound 1 (1, 3, 10, 20, 40, 80 and 160 mg) or placebo was orally administered under fasting conditions. Eight test subjects (6 in the active drug group and 2 in the placebo group) participated in each cohort. The concomitant use of all medications was prohibited from 28 days before hospitalization until discharge.

Inclusion Criteria:

Healthy Japanese adult male who is 20 to 45 years old, weighs 50 kg or more, and has a BMI within the range of 18.5-25.0 kg/m$^2$ at the time of obtaining consent for the clinical trial (the subject's parents and grandparents are Japanese).

Exclusion Criteria:

Those having cardiovascular disease, central nervous system disease, gastrointestinal disease, hematopoietic disease, renal failure, metabolic or endocrine disorder, severe allergy, asthma, hypoxemia, hypertension, convulsion or allergic rash at the time of this clinical trial. Those having abnormal clinical test values that suggest a clinically problematic disease at the time of screening.

Test schedule:

The test subjects were inspected and examined within 28-2 days and 1 day before administration of test compound 1, and eligibility was confirmed based on the above-mentioned inclusion criteria and exclusion criteria. The test subjects who met all inclusion criteria and did not violate any exclusion criteria were randomized and received oral administration of test compound 1 (1, 3, 10, 20, 40, 80, and 160 mg) at 9 o'clock at the clinical trial-conducting medical institution. They were fasted for 10 hr before administration and 4 hr after administration until lunch, and took lunch at 13:00 p.m. The blood was collected 0.5 hr before and 1, 2, 4, 6, 8, and 10 hr after administration of test compound 1.

Measurement of blood hormone concentration

The concentration of anterior pituitary hormone was measured using blood from the placebo administration group of all cohorts and the test subjects who received administration of 1 mg or 10 mg of test compound 1, at 0.5 hr before administration and 2, 4, 6, 8, and 10 hr after administration. The concentrations of growth hormone, follicle-stimulating hormone, luteinizing hormone, thyroid-stimulating hormone, adrenocorticotropic hormone, and prolactin in blood were measured using the Human Growth Hormone Quantikine ELISA Kit (manufactured by R&D Systems), Follicle-Stimulating Hormone (Human) ELISA Kit (manufactured by Cayman Chemical), Luteinizing Hormome (Human) ELISA Kit (manufactured by Cayman Chemical), U-TSH ELISA (manufactured by BIOCHECK), ACTH ELISA Kit (manufactured by MB Biosciences GmbH), and Human Prolactin Quantikine ELISA Kit (manufactured by R&D Systems) according to the protocol described in the package insert of each kit.

Results

The time course changes in the blood anterior pituitary hormone concentrations after oral administration of placebo (n=14) or test compound 1 at doses of 1 mg and 10 mg (n=6 for each) are shown in Fig. 1-6.
As is clear from Fig. 1-6, test compound 1 was shown to clearly enhance growth hormone secretion in humans. The test results clearly show that test compound 1 and SSTR5 antagonists represented thereby contribute to the prophylaxis and/or treatment of diseases that are improved by promoting growth hormone secretion.
Furthermore, test compound 1 did not substantially affect the secretion of other anterior pituitary hormones involving somatostatin, for example, follicle-stimulating hormone, luteinizing hormone, thyroid-stimulating hormone, adrenocorticotropic hormone, and prolactin. This shows that test compound 1 and SSTR5 antagonists represented thereby are expected to afford a selective growth hormone secretagogue action, without affecting secretion of all or some of anterior pituitary hormones such as follicle-stimulating hormone, luteinizing hormone, thyroid-stimulating hormone, adrenocorticotropic hormone, prolactin, and the like.
In the medical settings, treatments and the like for diseases caused by hyposecretion of growth hormone and/or relative insufficiency of growth hormone have been successfully performed, for example, by supplementing growth hormone from the outside (growth hormone replacement therapy). Regarding SSTR5 antagonists, the above clinical test results demonstrated that test Compound 1, which is a representative compound thereof, clearly has "the effect of promoting growth hormone secretion" in humans. Furthermore, it has been confirmed that an increase in blood growth hormone concentration caused by test compound 1 corresponds to the blood growth hormone concentration obtained by growth hormone replacement therapy for patients with pediatric growth hormone deficiency (J Clin Endocrinol Metab. 2017, 102(5):1578-1587). This clearly shows that SSTR5 antagonists are effective for the treatment and the like of pediatric growth hormone deficiency, short stature in Turner syndrome, short stature in chondrodystrophy, short stature in pediatric chronic renal failure, short stature in Prader-Willi syndrome, short stature in SGA, short stature in Noonan syndrome, adult growth hormone deficiency, which are diseases for which growth hormone replacement therapy is applied; cancer cachexia, short bowel syndrome, alopecia, skin wound, age-related hyposecretion of growth hormone, infertility, and the like, where supplementation of growth hormone improves pathology and symptoms, and those of ordinary skill in the art can easily understand the "effects on diseases" exerted by such SSTR5 antagonists based on the treatment status of diseases in medical settings and the above-mentioned clinical test results.

Furthermore, growth hormones have been externally supplemented to livestock animals in hopes of promoting their growth. Therefore, the above-mentioned test results of test compound 1 also clearly indicate that administration of SSTR5 antagonists to livestock animals and aquatic animals can be expected to promote the growth of animals due to their growth hormone secretion action. The effects of the SSTR5 antagonists can be easily understood by those skilled in the art.

[Industrial Applicability]

The present invention provides a growth hormone secretion promoter and the like and is useful, for example, in the pharmaceutical field.

This application is based on a patent application No. 2023-049987 filed in Japan (filing date: March 27, 2023), the contents of which are incorporated in full herein.

**Claims**

1. A growth hormone secretion promoter, comprising an SSTR5 antagonist as an active ingredient.

2. The growth hormone secretion promoter according to claim 1, which is an agent for the prophylaxis and/or treatment of a disease that is improved by promoting secretion of a growth hormone.

3. The growth hormone secretion promoter according to claim 2, wherein the disease that is improved by promoting secretion of a growth hormone is one or more diseases selected from pediatric growth hormone deficiency, short stature in Turner syndrome, short stature in chondrodystrophy, short stature in pediatric chronic renal failure, short stature in Prader-Willi syndrome, short stature in SGA, short stature in Noonan syndrome, adult growth hormone deficiency, cancer cachexia, short bowel syndrome, alopecia, skin wound, age-related hyposecretion of growth hormone, and infertility.

4. The growth hormone secretion promoter according to claim 2, wherein the disease that is improved by promoting secretion of a growth hormone is one or more diseases selected from pediatric growth hormone deficiency, short stature in Turner syndrome, short stature in chondrodystrophy, short stature in pediatric chronic renal failure, short stature in Prader-Willi syndrome, short stature in SGA, short stature in Noonan syndrome, and adult growth hormone deficiency.

5. The growth hormone secretion promoter according to claim 1, which is a growth promoter for an animal.

6. The growth hormone secretion promoter according to any one of claims 1 to 5, wherein the SSTR5 antagonist is selected from the compounds or salts thereof described in WO 2014/142363, WO 2015/052910, WO 2015/064083, WO 2010/056717, WO 2010/129729, WO 2011/146324, WO 2012/024183, WO 2016/205032, WO 2006/094682, WO 2006/128803, WO 2007/025897, WO 2007/110340, WO 2008/000692, WO 2008/019967, WO 2008/031735, WO 2008/122510, WO 2008/145524, WO 2008/148710, WO 2021/113362, WO 2021/113363, WO 2021/113368, WO 2023/125486, and European Journal of Medicinal Chemistry 264(2024)116017.

7. The growth hormone secretion promoter according to any one of claims 1 to 5, wherein the SSTR5 antagonist is selected from the compounds or salts thereof described in WO 2015/052910 and WO 2015/064083.

8. The growth hormone secretion promoter according to any one of claims 1 to 5, wherein the SSTR5 antagonist is selected from

6-(1-((2-cyclopropyl-5-ethoxy-4'-fluorobiphenyl-4-yl)methyl)piperidin-4-yl)-5-oxo-2-propyl-5,6,7,8-tetrahydro-1,6-naphthyridine-3-carboxylic acid,
6-(1-((6-cyclopropyl-3-ethoxy-2,4'-difluorobiphenyl-4-yl)methyl)piperidin-4-yl)-5-oxo-2-propyl-5,6,7,8-tetrahydro-1,6-naphthyridine-3-carboxylic acid,
6-(1-((2-cyclopropyl-5-ethoxy-2',4'-difluorobiphenyl-4-yl)methyl)piperidin-4-yl)-5-oxo-2-propyl-5,6,7,8-tetrahydro-1,6-naphthyridine-3-carboxylic acid,

6-(1-((6-cyclopropyl-3-ethoxy-2,4'-difluorobiphenyl-4-yl)methyl)piperidin-4-yl)-2-ethyl-5-oxo-5,6,7,8-tetrahydro-1,6-naphthyridine-3-carboxylic acid,

1-(1-((6-cyclopropyl-3-ethoxy-2,4'-difluorobiphenyl-4-yl)methyl)piperidin-4-yl)-3-ethyl-2-oxo-1,2-dihydropyridine-4-carboxylic acid,

1-(1-((6-cyclopropyl-2,4'-difluoro-3-isopropoxybiphenyl-4-yl)methyl)piperidin-4-yl)-3-methyl-2-oxo-1,2-dihydropyridine-4-carboxylic acid,

1-(1-((6-cyclopropyl-3-ethoxy-2,4'-difluorobiphenyl-4-yl)methyl)piperidin-4-yl)-3-methyl-2-oxo-1,2-dihydropyridine-4-carboxylic acid,

and salts thereof.

9. The growth hormone secretion promoter according to any one of claims 1 to 5, wherein the SSTR5 antagonist is 6-(1-((2-cyclopropyl-5-ethoxy-4'-fluorobiphenyl-4-yl)methyl)piperidin-4-yl)-5-oxo-2-propyl-5,6,7,8-tetrahydro-1,6-naphthyridine-3-carboxylic acid or a salt thereof.

10. A method for the prophylaxis and/or treatment of a disease that is improved by promoting secretion of a growth hormone, comprising administering an effective amount of an SSTR5 antagonist to a human or an animal in need of the administration.

11. An SSTR5 antagonist for use in the prophylaxis and/or treatment of a disease that is improved by promoting secretion of a growth hormone.

12. Use of an SSTR5 antagonist for the production of a medicament for the prophylaxis and/or treatment of a disease that is improved by promoting secretion of a growth hormone.

13. A method for promoting growth of an animal, comprising administering an effective amount of an SSTR5 antagonist to an animal in need of the administration.

14. An SSTR5 antagonist for use in promoting growth of an animal.

15. Use of an SSTR5 antagonist for the production of a growth promoter for an animal.

[Fig. 1]

changes in blood growth hormone concentration
(mean±standard deviation)

**Growth hormone**

[Fig. 2]

changes in blood follicle-stimulating hormone
concentration (mean±standard deviation)

**Follicle-stimulating hormone**

[Fig. 3]

changes in blood luteinizing hormone
concentration (mean±standard deviation)

Luteinizing hormone

[Fig. 4]

changes in blood thyroid-stimulating hormone
concentration (mean±standard deviation)

Thyroid-stimulating hormone

[Fig. 5]

changes in blood adrenocorticotropic hormone
concentration (mean±standard deviation)

**A d r e n o c o r t i c o t r o p i c   h o r m o n e**

[Fig. 6]

changes in blood prolactin hormone concentration
(mean±standard deviation)

**P r o l a c t i n**

## INTERNATIONAL SEARCH REPORT

| International application No. |
| --- |
| **PCT/JP2024/012098** |

**A. CLASSIFICATION OF SUBJECT MATTER**

*A61K 45/00*(2006.01)i; *A61K 31/444*(2006.01)i; *A61K 31/4375*(2006.01)i; *A61P 1/00*(2006.01)i; *A61P 5/00*(2006.01)i; *A61P 17/02*(2006.01)i; *A61P 17/14*(2006.01)i; *A61P 35/00*(2006.01)i
FI: A61K45/00; A61P5/00; A61P35/00; A61P1/00; A61P17/14; A61P17/02; A61K31/4375; A61K31/444

According to International Patent Classification (IPC) or to both national classification and IPC

**B. FIELDS SEARCHED**

Minimum documentation searched (classification system followed by classification symbols)

A61K45/00; A61K31/444; A61K31/4375; A61P1/00; A61P5/00; A61P17/02; A61P17/14; A61P35/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | JI, Jiuxiu et al. The effect of miR-6523a on growth hormone secretion in pituitary cells of Yanbian yellow cattle. Canadian Journal of Animal Science. 2020, vol. 100, no. 4, pp. 657-664, 10.1139/cjas-2019-0168 abstract, p. 662, right column to p. 663, left column, the last paragraph, fig. 1A, 2A, 4A, 5A | 1, 5, 13-15 |
| Y | | 1-15 |
| X | LUQUE, Raul M. et al. Identification of the somatostatin receptor subtypes (sst) mediating the divergent, stimulatory/inhibitory actions of somatostatin on growth hormone secretion. Endocrinology. 2006, vol. 147, no. 6, pp. 2902-2908, 10.1210/en.2005-1559 abstract, fig. 2 | 1 |
| Y | | 1-15 |
| X | WO 2021/230264 A1 (SCOHIA PHARMA INC.) 18 November 2021 (2021-11-18) claims, table 1 | 11, 14 |
| Y | | 1-15 |

☑ Further documents are listed in the continuation of Box C.  ☑ See patent family annex.

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **07 June 2024** | **18 June 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT

| International application No. |
|---|
| **PCT/JP2024/012098** |

### C. DOCUMENTS CONSIDERED TO BE RELEVANT

| Category* | Citation of document, with indication, where appropriate, of the relevant passages | Relevant to claim No. |
|---|---|---|
| X | JP 2016-531950 A (TAKEDA PHARMACEUTICAL COMPANY LIMITED.) 13 October 2016 (2016-10-13) example 4 | 11, 14 |
| Y | | 1-15 |
| Y | JP 2014-074004 A (NIJIMA, Shinichi) 24 April 2014 (2014-04-24) claims | 1-15 |
| P, X | 西崎晴庸 ほか, SCO-240, a small molecule antagonist for somatostatin receptor type 5 (SSTR5), stimulates growth hormone secretion in humans. 日本内分泌学会雑誌, 08 May 2023, vol. 99, no. 1, p. 310, ISSN: 0029-0661, (Folia Endocrinologica Japonica), non-official translation (NISHIZAKI, Harunobu) column O2-6-12 | 1, 2, 5, 10-15 |
| A | GILON, C. et al. A Backbone-Cyclic, Receptor 5-Selective Somatostatin Analogue: Synthesis, Bioactivity, and Nuclear Magnetic Resonance Conformational Analysis. Journal of Medicinal Chemistry JST Material no. D0102A ISSN: 0022-2623 CODEN, 1998, vol. 41, no. 6, pp. 919-929 abstract, fig. 9 | 1-15 |
| A | REN, Songguang et al. Functional association of somatostatin receptor subtypes 2 and 5 in inhibiting human growth hormone secretion. Journal of Clinical Endocrinology and Metabolism. 2003, vol. 88, no. 9, pp. 4239-4245, 10.1210/jc.2003-030303 | 1-15 |

Form PCT/ISA/210 (second sheet) (July 2022)

## INTERNATIONAL SEARCH REPORT
### Information on patent family members

International application No.

**PCT/JP2024/012098**

| Patent document cited in search report | | | Publication date (day/month/year) | Patent family member(s) | | | Publication date (day/month/year) |
|---|---|---|---|---|---|---|---|
| WO | 2021/230264 | A1 | 18 November 2021 | TW claims, table 1 | 202207929 | A | |
| JP | 2016-531950 | A | 13 October 2016 | WO example 4 | 2015/052910 | A1 | |
| | | | | US | 2015/0099777 | A1 | |
| | | | | EP | 3055309 | A1 | |
| | | | | CN | 105612158 | A | |
| JP | 2014-074004 | A | 24 April 2014 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014142363 A
- WO 2015052910 A
- WO 2015064083 A
- WO 2010056717 A
- WO 2010129729 A
- WO 2011146324 A
- WO 2012024183 A
- WO 2016205032 A
- WO 2006094682 A
- WO 2006128803 A
- WO 2007025897 A
- WO 2007110340 A
- WO 2008000692 A
- WO 2008019967 A
- WO 2008031735 A
- WO 2008122510 A
- WO 2008145524 A
- WO 2008148710 A
- WO 2021113362 A
- WO 2021113363 A
- WO 2021113368 A
- WO 2023125486 A
- JP 2023049987 A

### Non-patent literature cited in the description

- *Journal of Molecular Endocrinology*, 2014, vol. 53, R1-R19
- *J Clin Endocrinol Metab*, September 2003, vol. 88 (9), 4239-4245
- *Mol Endocrinol*, January 2003, vol. 17 (1), 93-106
- *European Journal of Medicinal Chemistry*, 2024, vol. 264, 116017
- Molecular Design. Development of Pharmaceuticals. Hirokawa Shoten, 1990, vol. 7, 163-198
- *J Clin Endocrinol Metab.*, 2017, vol. 102 (5), 1578-1587